(19) 

Europäisches Patentamt
European Patent Office
Office européen des brevets

(11) **EP 4 595 980 A1**

(12) **EUROPEAN PATENT APPLICATION**
published in accordance with Art. 153(4) EPC

(43) Date of publication:
06.08.2025 Bulletin 2025/32

(21) Application number: 23870981.0

(22) Date of filing: 28.09.2023

(51) International Patent Classification (IPC):
A61K 47/68 (2017.01)      A61K 38/17 (2006.01)
C07K 19/00 (2006.01)      A61K 39/395 (2006.01)
A61K 35/22 (2015.01)      A61P 13/12 (2006.01)

(52) Cooperative Patent Classification (CPC):
A61K 35/22; A61K 38/17; A61K 39/395;
A61K 47/68; A61P 13/12; C07K 19/00

(86) International application number:
PCT/CN2023/122388

(87) International publication number:
WO 2024/067756 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.09.2022 CN 202211208644

(71) Applicant: RemeGen Co., Ltd.
Shandong 264006 (CN)

(72) Inventors:
• YANG, Xiangdong
Jinan, Shandong 250012 (CN)

• GUO, Ling
Jinan, Shandong 250012 (CN)
• LI, Dengren
Jinan, Shandong 250012 (CN)
• FANG, Jianmin
Yantai, Shandong 264006 (CN)
• WANG, Wenxiang
Yantai, Shandong 264006 (CN)

(74) Representative: HGF
HGF Limited
1 City Walk
Leeds LS11 9DX (GB)

Remarks:
The complete document including Reference Table(s) and the Sequence Listing(s) can be downloaded from the EPO website

(54) **METHOD FOR TREATING MEMBRANOUS NEPHROPATHY WITH TACI-FC FUSION PROTEIN**

(57)    The present invention relates to a drug, a dosage scheme, an administration interval, and an administration mode for treating membranous nephropathy with a TACI-Fc fusion protein. Results show that the TACI-Fc fusion protein provided by the present invention shows relatively good clinical curative effect and good safety in the process of treating a patient with membranous nephropathy.

FIG. 1

EP 4 595 980 A1

## Description

### FIELD

The present disclosure relates to a TACI-Fc fusion protein drug for treating membranous nephropathy, and a dosage regimen, a dosing interval and an administration mode thereof.

### BACKGROUND

Membranous nephropathy (MN) is an antibody-mediated glomerular disease, which is characterized by the formation of immune deposits containing antigens, IgG and complement components beneath epithelial cells of glomerular capillary loops. The sublethal injury of epithelial cells leads to cellular simiplification and disruption of glomerular filtration barrier, resulting in proteinuria and other manifestations of nephrotic syndrome.

Clinically, membranous nephropathy can be divided into primary membranous nephropathy and secondary membranous nephropathy according to the etiology. The majority of membranous nephropathies are categorized as primary membranous nephropathies, also called as idiopathic membranous nephropathies, which is an organ-specific autoimmune disease that occurs in the absence of any identified cause or initiating event. About 20-30% of membranous nephropathies are categorized as secondary membranous nephropathies. Common causes include autoimmune diseases (e.g., lupus), viruses (e.g., hepatitis B virus, hepatitis C virus or syphilis), tumors, certain drugs (e.g. analgesics), toxins (e.g. exposure to heavy metals such as lead and mercury) and the like. Membranous nephropathy is one of the most common causes of nephrotic syndrome in non-diabetic adults, accounting for up to one-third of biopsy results of nephrotic syndrome cases. Its main clinical symptoms are oedema, proteinuria, hypoalbuminemia, hyperlipidaemia, and the like, which mainly affects the function of glomerular basement membrane, and also increases the risk of thrombosis, infection, cardiovascular disease and the like.

Currently, the global incidence of membranous nephropathy is approximately 1 case per 100,000 people per year. It remains unclear whether the incidence of membranous nephropathy varies by region or ethnicity, however, it can be confirmed that membranous nephropathy can occur in patients of all ages and ethnicities. In the United States, the incidence of membranous nephropathy is approximately 12 cases per million individuals per year, with an average onset age between 50 and 60 years. In China, the incidence of membranous nephropathy has been increasing over the past 20 years, and other studies have suggested that the high incidence of membranous nephropathy may be associated with worsening air pollution. According to statistics, among these patients with membranous nephropathy, nearly 60% of those who have not been treated for a long term have a decrease in kidney function, and 30-40% of them will eventually progress to end-stage renal disease within 10 years. Even being treated with immunosuppressants, 20%-40% of patients still show persistent heavy proteinuria during the course of the disease and will progress to end-stage renal disease within 10 years, which requires renal replacement or transplantation therapy.

The treatment of membranous nephropathy depends on its type and etiology. Currently, the treatment of membranous nephropathy mainly includes the following treatment methods: (1) conventional therapy: if the quantification of the 24-hour urine protein of the patient with membranous nephropathy is less than 3.5 g; or between 3.5 g and 8 g and the renal function is normal and there is no high-risk phenomenon, pril drugs and sartan drugs are usually used to reduce urine protein, and supportive therapy, such as diuresis, antihypertensive and anticoagulant treatment, is also given; (2) combination of hormone and immunosuppressant: if the quantification of the 24-hour urine protein of the patient with membranous nephropathy is greater than 3.5 g accompanied by renal function decrease; or the urine protein is greater than 8 g, a combination therapy of hormone and immunosuppressant is adopted for treatment; and (3) monoclonal antibody drugs (such as rituximab).

Table 1 Treatment regimens for membranous nephropathy recommended in 2021 KDIGO Guideline

| Cyclophosphamide (cyclical administration) | • Intravenous injection of methylprednisolone 1 g for 3 consecutive days in the first few days of the $1^{st}$ , $3^{rd}$, and $5^{th}$ month, <br> • Oral prednisone 0.5 mg/kg/day, <br> • Oral cyclophosphamide 2.5 mg/kg body weight/day in the $2^{nd}$, $4^{th}$ and $6^{th}$ month |
|---|---|
| Cyclophosphamide (continuous administration) | • Intravenous injection of methylprednisolone 1 g for 3 consecutive days in the first few days of the $1^{st}$ , $3^{rd}$, and $5^{th}$ month, <br> • Oral prednisone 0.5 mg/kg every other day, for 1-6 months, then gradually tapered, <br> • Oral cyclophosphamide 1.5 mg/kg body weight/day for 16 months |
| Rituximab | • Rituximab 1000 mg IV twice within 2 weeks <br> • Rituximab 375 mg/$m^2$ body surface area weekly, total 1-4 times |

(continued)

| Tacrolimus | • Tacrolimus 0.05-0.1 mg/kg body weight/day, aiming at 3-8 ng/ml blood drug concentration maintained for 12 months |
|---|---|
| Cyclosporine | • Cyclosporine 3.5 mg/kg body weight/day, aiming at 125-225 mg/ml blood drug concentration |

The 2021 KDIGO Guideline recommend five treatment regimens for membranous nephropathy (see Table 1), which are cyclophosphamide in cyclical administration or continuous administration, rituximab, tacrolimus and cyclosporine, respectively. Wherein: 1) the cyclical or continuous administration of cyclophosphamide must be combined with the administration of hormonal drugs (e.g., prednisone), while hormonal drugs have significant side effects and cyclophosphamide has also adverse reactions such as reproductive toxicity, myelosuppression, infection and haemorrhagic cystitis. Moreover, from the perspective of application reality, after treating using the hormone + cyclophosphamide regimen, there are still nearly half of the patients cannot be relieved, and once the patients who get relief stop taking the drug, the disease is likely to relapse. (2) Tacrolimus and cyclosporine are calcineurin inhibitors (CNIs), which belong to a broad category of immunosuppressive drugs. For patients who are unwilling to receive glucocorticoid + cyclophosphamide therapy or have contraindications to treatment, CNIs can be used for treatment. Wherein, the cyclosporine therapy has the advantage of rapid onset of action, with better efficacy and fewer and milder adverse effects compared to cyclophosphamide. Its disadvantages include an overall remission rate no higher than that of cyclophosphamide regimen and a significantly higher short-term recurrence rate compared to cyclophosphamide. Although the immunosuppressive potency of tacrolimus is 10-100 times higher than that of cyclosporine, it also has similar shortcomings to cyclosporine, i.e., tacrolimus also has the problem of a high recurrence rate. Additionally, a prominent adverse effect of tacrolimus is that it leads to abnormal glucose tolerance and new-onset diabetes. (3) Currently, rituximab alone or in combination with a calcineurin inhibitor is recommended for the initial treatment of primary membranous nephropathy with intermediate, high, or very high risk, with a remission rate of 67% for membranous nephropathy. In addition, rituximab is applicable for patients with membranous nephropathy who are either positive or negative for anti-PLA2R antibodies. However, rituximab is expensive, has a low patient accessibility, and has severe infusion reactions. Various studies have reported that the incidence of infusion-related reactions associated with rituximab ranges from 26% to 85%, and in patients with hematological neoplasms, rare adverse events such as reactivation of hepatitis B virus and severe skin reactions (e.g., toxic epidermal necrolysis and Stevens-Johnson syndrome) caused by rituximab have also been reported (Reference 1: Alsharhan L, Beck LH Jr. Membranous Nephropathy: Core Curriculum 2021. Am J Kidney Dis.2021 Mar; 77(3): 440-453). In general, the clinical course of membranous nephropathy is often indolent, and existing treatments are toxic. Existing treatment options have certain limitations and risks, and their therapeutic effects are not always satisfactory. However, from the clinical application perspective, if the combination therapy of hormone and immunosuppressant is ineffective, monoclonal antibody drugs (such as rituximab) are usually used for treatment. Therefore, the development of new biological drugs (e.g. monoclonal antibody drugs) has become an urgent need for the treatment of membranous nephropathy.

Table 2 Biological drugs approved and in Phase III clinical trial for treating membranous nephropathy

| Drug name | Target | Research institute | Highest research and development stage |
|---|---|---|---|
| Rituximab | CD20 | Roche | Approved for marketing |
| Obinutuzumab | CD20 | Glycart Biotechnology (Roche) | Approved for marketing |
| MIL62 | CD20 | Mabworks | Clinical phase III |
| Felzartamab | CD38 | TJ Biopharma | Clinical phase III |

As of September 22, 2022, only two biological drugs for membranous nephropathy have been approved for marketing in the world, which are rituximab and obinutuzumab, respectively. In addition, there are two other drugs in phase III clinical research stage (see Table 2). Among these drugs, although rituximab was included in the recommended treatment regimen for membranous nephropathy of KDIGO Guidelines in 2021, it still faces problems such as a low remission rate, high infusion reactions, severe adverse reactions and a high price. Although obinutuzumab, which also targets CD20, can achieve certain therapeutic effects on patients with membranous nephropathy who have failed treatments clinically, the relevant therapeutic effect is only partial remission rather than complete remission, because it cannot completely eliminate antibodies and immune complexes, the basement membrane of the kidney is still damaged, and some urine protein will still leak out. In addition, as obinutuzumab is more cytotoxic compared to rituximab and has a stronger ability to consume B cells, it may face greater challenges in terms of adverse reactions (Reference 2: Hudson R, Rawlings C, Mon SY, et al. Treatment resistant M-type phospholipase A2 receptor associated membranous nephropathy responds to obinutuzumab: a report of two cases. BMC Nephrol. 23,134(2022)). Therefore, there is a huge unmet clinical need in the field of membranous nephropathy treatment, both in China and globally.

SUMMARY

The present disclosure discovers a TACI-Fc fusion protein having significant therapeutic effects in treating patients with membranous nephropathy.

Specifically, the present disclosure provides a method of treating membranous nephropathy, and the method comprises administering a therapeutically effective amount of a TACI-Fc fusion protein to a patient with membranous nephropathy.

Specifically, the present disclosure further provides a method of treating a patient with membranous nephropathy who has already received a treatment regimen for membranous nephropathy, and the method comprises (1) determining whether the patient has already received a treatment regimen for membranous nephropathy, and (2) if that the patient has previously received a treatment for membranous nephropathy, administering a therapeutically effective amount of a TACI-Fc fusion protein to the patient with membranous nephropathy.

Specifically, the present disclosure further provides use of a TACI-Fc fusion protein in the manufacture of a medicament for treating a patient with membranous nephropathy.

Further, the TACI-Fc fusion protein described in any one of the foregoing items comprises (i) a TACI extracellular region or a fragment thereof binding to Blys and/or APRIL; and (ii) a fragment of human immunoglobulin constant region.

Preferably, the TACI extracellular region or the fragment thereof comprises an amino acid sequence set forth in SEQ ID NO: 1.

## SEQ ID NO: 1

| | |
|---|---|
| SRVDQEERFP QGLWTGVAMR SCPEEQYWDP LLGTCMSCKT ICNHQSQRTC AAFCRSLSCR | 60 |
| KEQGKFYDHL LRDCISCASI CGQHPKQCAY FCENKLRSPV NLPPEL | 106 |

Preferably, the human immunoglobulin is IgG1.

Further, the fragment of human immunoglobulin constant region comprises an amino acid sequence set forth in SEQ ID NO: 2 or an amino acid sequence having at least 90%, at least 91%, at least 92%, at least 93%, at least 95%, at least 96%, at least 97%, at least 98%, or at least 99% identity to SEQ ID NO: 2.

## SEQ ID NO: 2

| | |
|---|---|
| DKPHTCPLCP APELLGGPSV FLFPPKPKDT LMISRTPEVT CVVVDVSHED PEVKFNWYVD | 60 |
| GVEVHNAKTK PREEQYNSTY RVVSVLTVLH QDWLNGKEYK CKVSNKALPA PIEKTISKAK | 120 |
| GQPREPQVYT LPPSRDELTK NQVSLTCLVK GFYPSDIAVE WESNGQPENN YKATPPVLDS | 180 |
| DGSFFLYSKL TVDKSRWQQG NVFSCSVMHE ALHNHYTQKS LSLSPGK | 227 |

Further, the fragment of human immunoglobulin constant region comprises one or more modifications of amino acid at positions 3, 8, 14, 15, 17, 110, 111 or 173 of SEQ ID NO: 2.

Further, the modification is substitution, deletion or insertion of an amino acid.

Further, the substitution is selected from the group consisting of P3T, L8P, L14A, L15E, G17A, A110S, P111S and A173T.

Preferably, the fragment of human immunoglobulin constant region comprises an amino acid sequence set forth in SEQ ID NO: 3.

SEQ ID NO: 3

DKTHTCPPCP APEAEGAPSV FLFPPKPKDT LMISRTPEVT CVVVDVSHED PEVKFNWYVD    60

GVEVHNAKTK PREEQYNSTY RVVSVLTVLH QDWLNGKEYK CKVSNKALPS SIEKTISKAK    120

GQPREPQVYT LPPSRDELTK NQVSLTCLVK GFYPSDIAVE WESNGQPENN YKTTPPVLDS    180

DGSFFLYSKL TVDKSRWQQG NVFSCSVMHE ALHNHYTQKS LSLSPGK    227

Preferably, the TACI-Fc fusion protein has an amino acid sequence set forth in SEQ ID NO: 4.

SEQ ID NO: 4

SRVDQEERFP QGLWTGVAMR SCPEEQYWDP LLGTCMSCKT ICNHQSQRTC AAFCRSLSCR    60

KEQGKFYDHL LRDCISCASI CGQHPKQCAY FCENKLRSPV NLPPELDKTH TCPPCPAPEA    120

EGAPSVFLFP PKPKDTLMIS RTPEVTCVVV DVSHEDPEVK FNWYVDGVEV HNAKTKPREE    180

QYNSTYRVVS VLTVLHQDWL NGKEYKCKVS NKALPSSIEK TISKAKGQPR EPQVYTLPPS    240

RDELTKNQVS LTCLVKGFYP SDIAVEWESN GQPENNYKTT PPVLDSDGSF FLYSKLTVDK    300

SRWQQGNVFS CSVMHEALHN HYTQKSLSLS PGK    333

Preferably, the TACI-Fc fusion protein is telitacicept.
Further, the TACI-Fc fusion protein is administered at a single dose of about 0.1 to 10 mg/kg, further including 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.6, 6.6, 6.7, 6.8, 6.9, 7.0, 7.1, 7.2, 7.3, 7.4, 7.7, 7.6, 7.7, 7.8, 7.9, 8.0, 8.1, 8.2, 8.3, 8.4, 8.8, 8.6, 8.7, 8.8, 8.9, 9.0, 9.1, 9.2, 9.3, 9.4, 9.9, 9.6, 9.7, 9.8, 9.9 and 10 mg/kg.
Further, the TACI-Fc fusion protein is administered at a single dose of 160 to 240 mg, further preferably 160 mg, 170mg, 180mg, 190mg, 200mg, 210mg, 220mg, 230mg or 240mg.
Further, a method for detecting the content of the above fusion protein is UV-visible spectrophotometry, wherein according to the fact that the protein has a maximum UV absorption at 280 nm, the absorbance value of the telitacicept sample is measured at this wavelength. After correcting the absorbance at 320 nm, the absorbance value measured at 280 nm is proportional to the protein concentration, and the protein concentration is calculated according to Lambert-Beer law to determine the protein content. The formula for calculating the protein content is as follows:

$$\text{Protein content (mg/ml)} = \underline{\text{(or (corrected))}} * \text{sample dilution multiple}$$

In the formula, $\varepsilon$ represents the extinction coefficient value of telitacicept in $(mg/ml)^{-1} \cdot cm^{-1}$.
$A_{280}$ represents the average value of absorbance of sample solutions at 280 nm.
$A_{280}$ (corrected) represents the average value of absorbance of sample solutions at 280 nm after correction.

Further, the TACI-Fc fusion protein is administered 2-4 times at an interval of one month. That is, the administration frequency of the TACI-Fc fusion protein is 2 times per month or 3 times per month or 4 times per month.
Further, the TACI-Fc fusion protein is administered at a frequency of once a week.
Further preferably, the administration lasts for about 2 to 50 weeks. Still further preferably, the administration lasts for 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 weeks.
Further preferably, the TACI-Fc fusion protein is administered subcutaneously, intramuscularly or intravenously, at a position of preferably thigh, abdomen or upper arm. In some specific embodiments, the TACI-Fc fusion protein is administered by subcutaneous, intramuscular or intravenous injection.
Further preferably, the TACI-Fc fusion protein is injected at the same site or at different sites per injection. In some specific embodiments, the TACI-Fc fusion protein is injected at the same site per injection. In some other specific embodiments, the TACI-Fc fusion protein is injected at different sites per injection.

Further, the membranous nephropathy is primary membranous nephropathy or secondary membranous nephropathy.

Further, the membranous nephropathy is PLA2R-positive or PLA2R-negative.

Further, the patient is an adult patient or a child patient.

Further, the patient has previously received a treatment regimen for membranous nephropathy.

Further, the treatment regimen for membranous nephropathy includes conventional therapy of membranous nephropathy, combination therapy of hormone and immunosuppressant, and monoclonal antibody drugs therapy.

Further, the treatment regimen for membranous nephropathy includes: cyclical administration of cyclophosphamide in combination with a hormone drug to the patient, or continuous administration of cyclophosphamide in combination with a hormone drug to the patient, or administration of tacrolimus to the patient, or administration of cyclosporine to the patient, or administration of rituximab to the patient, or administration of obinutuzumab to the patient, or administration of MIL62 to the patient, or administration of felzartamab to the patient.

The TACI-Fc fusion protein provided by the present disclosure shows satisfactory clinical effects and a good safety in the treatment of patients with membranous nephropathy.

**BRIEF DESCRIPTION OF DRAWINGS**

FIG. 1 shows changes in albumin of subjects during the treatment in Example 1.

FIG. 2 is a diachronic analysis of the change rate of immunoglobulin G (IgG) level of the subjects during the treatment compared with baseline in Example 1.

FIG. 3 is a diachronic analysis of the change rate of immunoglobulin M (IgM) level of the subjects during the treatment compared with baseline in Example 1.

FIG. 4 is a diachronic analysis of the change rate of immunoglobulin A (IgA) level of the subjects during the treatment compared with baseline in Example 1.

**DETAILED DESCRIPTION**

Unless otherwise defined, all technical and scientific terms used herein have the same meaning as commonly understood by one of ordinary skill in the art. For definitions and terms in the art, those skilled can make a specific reference to Current Protocols in Molecular Biology (Ausubel).

The three-letter codes and one-letter codes of amino acids used in the present disclosure are as described in J. biol. chem, 243, p3558 (1968).

The term "TACI" in the present disclosure refers to transmembrane activator and CAML interactor, which is a member of the tumor necrosis factor receptor superfamily. The term "BLys" in the present disclosure refers to B lymphocyte stimulator, which is a member of the TNF ligand superfamily existing in two forms of membrane-bound and soluble forms, specifically expressed on the surface of bone marrow cells, and selectively stimulates the proliferation of B lymphocytes and the production of immunoglobulin. The term "APRIL" (a proliferation-inducing ligand) in the present disclosure refers to a tumor necrosis factor (TNF) analogue, which can stimulate the proliferation of primitive B cells and T cells in the body, promote the accumulation of B cells and increase their content in spleen. APRIL can specifically bind to TACI and BCMA, and the binding can prevent APRIL from binding to B cells and thus inhibit the proliferative response of primitive B cells stimulated by APRIL. Moreover, APRIL can compete with BLys for binding to receptors (BCMA and TACI).

The term "TACI-Fc fusion protein" involved in the present disclosure refers to transmembrane activator, calcium regulator and cyclophilin ligand interactor (TACI)-immunoglobulin fusion protein (i.e., TACI-Fc fusion protein). The TACI-immunoglobulin fusion protein provided by the present disclosure comprises: (i) a TACI extracellular region or a fragment thereof binding to Blys and/or APRIL; and (ii) a fragment of human immunoglobulin constant region.

For the term "TACI extracellular region or a fragment thereof binding to Blys and/or APRIL", specific reference can be made to the TACI extracellular domain and the specific fragment of TACI extracellular domain capable of interacting with TACI ligands disclosed in US Patent Nos. 969,102, 6,316,222 and 6,500,428 and US Patent Application Nos. 09/569,245 and 09/627,206, the contents of which are incorporated herein by reference, or the fragment of amino acids 13-118 of TACI extracellular domain disclosed in Chinese Patent Publication No. CN101323643A.

In the term "fragment of human immunoglobulin constant region", the immunoglobulin is preferably IgG1, which may comprise a heavy chain constant region, for example, heavy chain constant region of human. The preferred "fragment of human immunoglobulin constant region" of the present disclosure is an amino acid fragment comprising a portion of the hinge region domain, a CH2 domain and a CH3 domain. In some more preferred embodiments, the "fragment of human immunoglobulin constant region" described in the present disclosure has an amino acid sequence set forth in SEQ ID NO: 2, or an amino acid sequence having at least 90%, at least 91%, at least 92%, at least 93%, at least 95%, at least 96%, at least 97%, at least 98%, or at least 99% identity to SEQ ID NO: 2. In some more preferred embodiments, the "fragment of human immunoglobulin constant region" has an amino acid sequence set forth in SEQ ID NO:3.

The term "treatment" involved in the present disclosure is related to a given disease or condition, including but not limited to: inhibiting the disease or condition, for example, preventing the development of the disease or condition; alleviating the disease or condition, for example, causing the regression of the disease or condition; or ameliorating the symptoms caused by the disease or condition, for example, alleviating, preventing or treating the symptoms of the disease or condition.

The term "amino acid" involved in the present disclosure is understood in the broadest sense, and it is a general term for a class of organic compounds containing amino and carboxyl. Preferably, the amino acid involved in the present disclosure is the main unit of proteins constituting living organisms, which includes, but is not limited to: glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine and histidine.

The three-letter codes and one-letter codes of amino acids used in the present disclosure are as described in J. biol. chem, 243, p3558 (1968). There are many ways of numbering amino acid positions, such as Kabat numbering system, EU numbering system, and sequence numbering. In the present disclosure, the amino acid positions are numbered using "sequence numbering", for example, the "position 3, 8, 14, 15, 17, 110, 111 or 173 of SEQ ID NO: 2" in the present disclosure refers to the 3rd amino acid, the 8th amino acid, and so on of SEQ ID NO: 2. For example, the "P3T" in the present disclosure refers to the mutation of the 3rd amino acid of SEQ ID NO:2 from the previous "P" to "T", and "L8P" refers to the mutation of the 8th amino acid of SEQ ID NO:2 from the previous "L" to "P", and so on.

As an alternative embodiment, the immunoglobulin constant region provided by the present disclosure can be introduced with one or more amino acid modifications, such as substitution (i.e., mutation), addition (i.e., insertion) or deletion (i.e., absence).

The term "telitacicept" (or called as Tai'ai, which may be used interchangeably in the present disclosure) used in the present disclosure refers to a TACI-Fc fusion protein, having an INN name of Telitacicept and an amino acid sequence set forth in SEQ ID NO: 4, or referring to https://extranet.who.int/soinn/mod/page/viewphp? id = 137&inn_n= 10932.

The TACI-Fc fusion protein of the present disclosure may be administered by any one of various routes, which include, but are not limited to oral administration, intravenous injection, intramuscular injection, intraarterial injection, intramedullary injection, intraperitoneal injection, intrathecal injection, intra-cardiac and -cerebral administration, transdermal administration, transdermal administration, topical administration, subcutaneous administration, intranasal administration, enteral administration, sublingual administration, vaginal administration, rectal administration, etc.

The term "membranous nephropathy" used in the present disclosure refers to a series of diseases sharing a common histo-pathological pattern, i.e., the presence of immunoglobulins and immune deposits containing complement in the subepithelial space.

The term "idiopathic membranous nephropathy" used in the present disclosure refers to an organ-specific autoimmune disease that occurs in the absence of any identified cause or initiating event, including, but not limited to, PLA$_2$R-associated idiopathic membranous nephropathy, THS7A-associated idiopathic membranous nephropathy, NELL-1-associated idiopathic membranous nephropathy, Sema3B-associated idiopathic membranous nephropathy, and other associated idiopathic membranous nephropathies.

The term "secondary membranous nephropathy" used in the present disclosure refers to a glomerular disease with clear systemic disorder involving nephropathy and pathological changes to membranous nephropathy. The cause of the secondary membranous nephropathy includes an immunological disease (e.g., systemic lupus erythematosus, type 1 diabetes mellitus, rheumatoid arthritis, Hashimoto's thyroiditis, Graves' disease, mixed connective tissue disease, allergic purpura, primary biliary cirrhosis, small bowel enteropathy syndrome, anti-glomerular basement membrane nephritis, ANCA-associated crescentic nephritis, graft-versus-host disease, bone marrow and stem cell transplantation, etc.), infection and parasitic diseases (e.g., HBV, HCV, syphilis, filariasis, cysticercosis, schistosome, plasmodium and leprosy), and drugs and toxins (e.g. gold preparation, penicillamine, non-steroidal anti-inflammatory drugs, mercury, captopril, formaldehyde, hydrocarbons) and others (e.g. tumors and kidney transplantation).

The term "PLA2R-positive" used in the present disclosure means that PLA2R is detected in the blood of the patient, i.e., the patient is serologically positive for PLA2R.

The term "PLA2R-negative" used in the present disclosure means that PLA2R is not detected in the blood of the patient.

The term "conventional therapy of membranous nephropathy" used in the present disclosure exemplarily means that if the quantification of the 24-hour urine protein of the patient with membranous nephropathy is less than 3.5 g; or between 3.5g and 8g the renal function is normal and there is no high-risk phenomenon, pril drugs and sartan drugs are usually used to reduce urine protein, and supportive therapy, such as diuresis, antihypertensive and anticoagulant treatment is also given.

The term "combination therapy of hormone and immunosuppressant" used in the present disclosure exemplarily means that if the quantification of the 24-hour urine protein of the patient with membranous nephropathy is greater than 3.5 g accompanied by renal function decrease; or the urine protein is greater than 8 g, a combination therapy with hormone and immunosuppressant is adopted for treatment, wherein the immunosuppressant includes, but is not limited to tacrolimus and cyclosporine.

The term "monoclonal antibody drugs therapy" used in the present disclosure exemplarily refers to therapy using, but not limited to, rituximab, as recommended in the 2021 KDIGO Guideline, for example, twice administration of rituximab 1000 mg via vein within 2 weeks; rituximab 375 mg/m$^2$ body surface area weekly, total 1-4 times.

The term "cyclical administration of cyclophosphamide in combination with hormone drugs" used in the present disclosure refers to therapy as recommended in the 2021 KDIGO Guideline, intravenous injection of methylprednisolone 1 g for 3 consecutive days in the first few days of the 1st, 3rd, and 5th month; oral prednisone 0.5 mg/kg/day; and oral cyclophosphamide 2.5 mg/kg body weight/day in the 2nd, 4th and 6th month.

The term "continuous administration of cyclophosphamide in combination with hormone drugs" used in the present disclosure refers to therapy as recommended in the 2021 KDIGO Guideline, intravenous injection of methylprednisolone 1 g for 3 consecutive days in the first few days of the 1st , 3rd, and 5th month; oral prednisone 0.5 mg/kg every other day, for 1-6 months, then gradually tapered; and oral cyclophosphamide 1.5 mg/kg body weight/day for 16 months.

The embodiments of the present disclosure will be described in detail below in conjunction with examples. However, those skilled in the art will understand that the following examples are only intended to illustrate the present disclosure, and should not be construed as limiting the scope of the present disclosure.

Example 1 Clinical trial of telitacicept for the treatment of membranous nephropathy

1. Research methods

This prospective, single-centre, unblinded, single-arm clinical trial primarily aimed to evaluate the efficacy and safety of telitacicept in the treatment of patients with membranous nephropathy, and to explore potential side effects of telitacicept in the treatment of patients with refractory membranous nephropathy. It was planned to enroll 30 patients with membranous nephropathy (12 patients had been enrolled for mid-term analysis) as subjects, receiving subcutaneous injection of 160 mg of telitacicept once a week for 48 weeks.

2. Enrolled patients

Subject inclusion criteria

• Patients diagnosed with primary membranous nephropathy by pathologically renal puncture and also positive for PLA2R antibodies;

• Age 18-70 years;

• Maintain 24-hour urine protein (UTP) ≥ 3.5 g on the basis of adequate ACEI/ARB drug treatment for at least 3 months prior to enrollment;

• Stable blood pressure, ≤ 140/90 mmHg;

• Estimated glomerular filtration rate (eGFR) ≥45 mL/min/1.73m$^2$;

• Agree to maintain a stable diet and sodium intake during the study period;

• Agree and sign the informed consent form.

Subjects were also excluded according to the following criteria:

• Unstable urine protein quantification within the last 2 months, with a fluctuation of 24-hour urine protein quantification >2 g/d;

- Combined with chronic liver disease or liver enzymes exceeding the upper limit of normal by more than 3 times;

- New onset of cardiovascular or cerebrovascular disease (such as acute coronary syndrome, heart failure, cerebral infarction and brain stroke) within 3 months;

- Uncontrolled severe hypertension;

- Malignant tumor;

- Patients with severe infections;

- Patients who have previously used telitacicept, or are allergic to telitacicept or other contraindication;

- Pregnancy or lactation;

- Life expectancy less than 6 months;

- Currently participating in other clinical studies;

- Other situations as determined to be unsuitable for participation in this study by the investigator.

3. Endpoint indicators

(1) Primary endpoint indicator

| Number | Indicator | Evaluation time | Endpoint |
|---|---|---|---|
| 1 | Excretion level of 24-hour urine protein at week 48 | Week 48 | Complete remission: urine protein content below 0.3 g/24 h |
| | | | Partial remission: urine protein content of 0.3-3.5g/24h, or 50% reduction in urine protein compared with baseline |

(2) Secondary endpoint indicators

| Number | Indicators | Evaluation time |
|---|---|---|
| 1 | Changes in 24-hour urine protein content (or urine protein/creatinine ratio and urine albumin/creatinine ratio) | Week 0, 4, 8, 12, 16, 20, 24, 36 and 48 |
| 2 | Change in estimated glomerular filtration rate (eGFR) | Week 0, 4, 8, 12, 16, 20, 24, 36 and 48 |
| 3 | Changes in serum albumin | Week 0, 4, 8, 12, 16, 20, 24, 36 and 48 |
| 4 | Changes in immunoglobulins IgA, IgG, and IgM | Week 0, 4, 8, 12, 16, 20, 24, 36 and 48 |
| 5 | Side effects of drug | / |

5. Main results of mid-term analysis

5. 1 Primary efficacy endpoints

The primary endpoint of this study is the excretion level of 24-hour urine protein at week 48. Wherein complete remission was indicated by a 24-hour urine protein content of less than 0.3 g/24 h, and partial remission was indicated by a 24-hour urine protein content between 0.3-3.5 g/24 h or a 50% reduction in urine protein compared with baseline. At week 48 or the end of treatment, there were 10 subjects with a reduction in urine protein compared with baseline, accounting for 83.3% of the current enrolled population. Among them, a total of 5 subjects had a 24-hour urine protein content between 0.3-3.5 g/24 h, and 3 subjects had a reduction of more than 50% compared with baseline (the reduction percent compared with baseline was 70.86%, 77.42% and 94.17%, respectively). As a result, 5 subjects achieved partial remission in this trial, accounting 42% of the total number of enrolled population. The 24-hour urine protein indicator of the subjects at baseline and at week 48 or the end of treatment, and the reduction percent compared with baseline are detailed in Table 2.

Table 2 24-hour urine protein content and reduction percent compared with baseline

| Subject | 24-hour urine protein (baseline) Unit: ml/min | 24-hour urine protein (at week 48 or end of treatment) Unit: ml/min | reduction percent compared with baseline |
|---|---|---|---|
| Subject 1 | 4.53 | 1.32 | 70.86% |
| Subject 2 | 10.23 | 2.31 | 77.42% |
| Subject 3 | 7.19 | 0.419 | 94.17% |
| Subject 4 | 4.44 | 2.97 | / |
| Subject 5 | 3.72 | 3.02 | / |

5.2 Secondary efficacy endpoints

5.2.1 Analysis of change in urine protein/creatinine ratio and urine albumin/creatinine ratio

In this study, the average urine protein/creatinine ratio (UPCR) and urine albumin creatinine ratio (UACR) of the subjects decreased compared with the baseline. The decrease had statistical significance compared with the baseline.

5.2.2 Analysis of change in estimated glomerular filtration rate (eGFR)

In this study, 4 subjects showed an increase in the indicator of estimated glomerular filtration rate (eGFR) after the end of medication, and the subject with the highest increase in eGFR increase from 47.9 mL/min/1.73m$^2$ to 62.46 mL/min/1.73m$^2$, with the increase percent of 30.4%.

5.2.3 Diachronic analysis of serum albumin

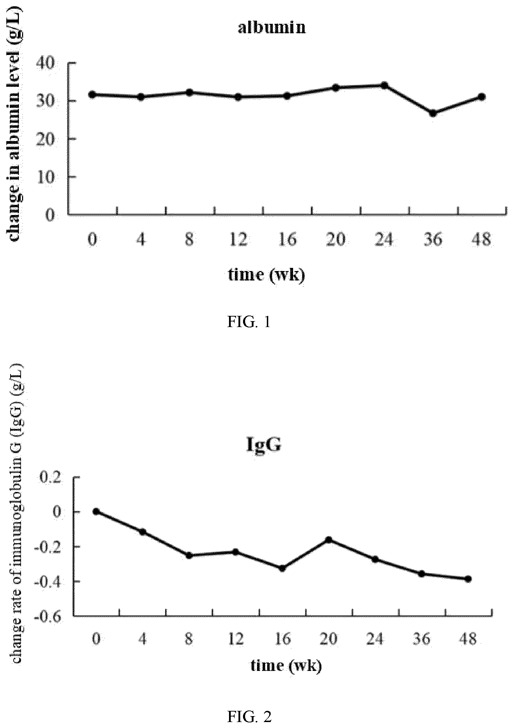

In this study, the average albumin level showed a reduction during the treatment process. After the end of medication, the average serum albumin level of enrolled subjects decreased by 0.59 g/L compared with the baseline. The diachronic change value of albumin is shown in FIG. 1.

5.2.4 Immunological indicators (IgG, IgM and IgA)

5.2.4.1 Diachronic analysis of immunoglobulin G (IgG)

Compared with the baseline, after 48 weeks of medication, the change rate of average IgG level of enrolled subjects showed an overall downward trend. The average IgG level was decreased by 2.60 g/L (the decrease rate was 38.8%). The diachronic change rate of average IgG level of the enrolled subjects is shown in FIG. 2.

5.2.4.2 Diachronic analysis of immunoglobulin M (IgM)

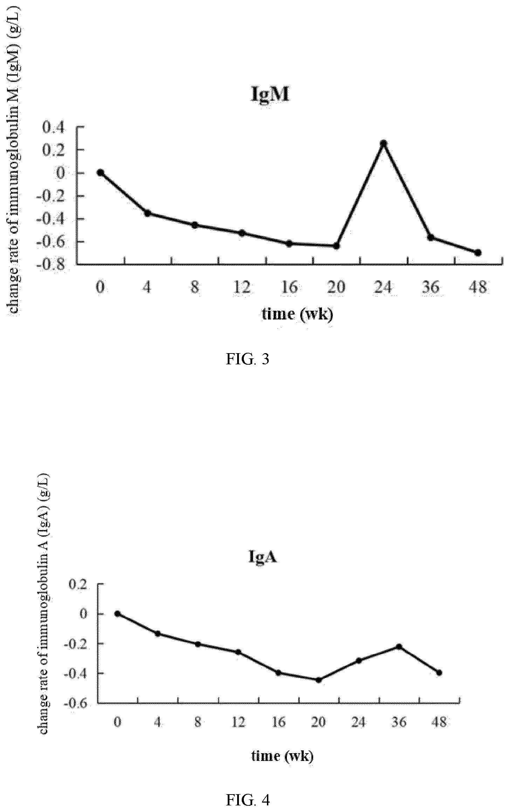

Compared with the baseline, the change rate of average IgM level of enrolled subjects also showed an overall downward trend. After 48 weeks of medication, the average IgM level was decreased by 0.708 g/L (the decrease rate was 70.1%). The diachronic change rate of average IgM level of the enrolled subjects is shown in FIG. 3.

5.2.4.3 Diachronic analysis of immunoglobulin A (IgA)

Compared with the baseline, the change rate of average IgA level of the enrolled subjects also showed an overall downward trend. After 48 weeks of medication, the average IgA level was decreased by 0.717 g/L (the decrease rate was 39.7%). The diachronic change rate of average IgA level of the enrolled subjects is shown in FIG. 4.

5.3 Efficacy results

In this study, the existing data showed that telitacicept exhibited consistently improved clinical efficacy in the treatment of patients with primary membranous nephropathy, and the average urine protein/creatinine ratio (UPCR) and urine albumin/creatinine ratio (UACR) of the subjects decreased compared with the baseline. The decrease had statistical significance compared with the baseline. After the administration of telitacicept, the average level of IgG, IgA and IgM of subjects were significantly reduced compared with the baseline.

5.4 Safety results

In this study, the current results showed that once-weekly administration of 160 mg of telitacicept had a good safety for the treatment of patients with primary membranous nephropathy. The severity of adverse events/adverse reactions was mild (CTCAE grade 1), and no grades 2, 3, 4, and 5 adverse events/adverse reactions occurred. There were no adverse events/adverse reactions that resulted in the withdrawal of subjects from the study or death occurred, and no serious adverse reactions occurred. Two subjects experienced adverse events during the study period, one of whom had abdominal pain and vomiting, but the occurrence of this adverse event/adverse reaction was not considered to be related to telitacicept, and the other had symptoms of skin pruritus which was relieved without drug administration after 2-3 days.

5.5 Conclusion

Based on the above results and analysis, in this study, the current trial data showed that telitacicept exhibited good clinical efficacy and safety in the treatment of patients with membranous nephropathy.

The above description is only for preferred embodiments by way of example only and without limitation to the combination of features necessary for carrying the present disclosure into effect. The headings provided herein are not intended to limit the various embodiments of the present disclosure. Terms such as "including", "comprising" and "containing" are not intended to be limiting. In addition, unless otherwise indicated, the singular form "a", "an", or "the" includes plural references, as well as "or" means "and/or". Unless otherwise defined herein, all technical and scientific terms used herein have the same meaning as commonly understood by those skilled in the art.

All publications and patents mentioned in the present application are incorporated herein by reference. Without departing from the scope and spirit of the present disclosure, various modifications and variations of the described method and composition of the present disclosure will be apparent to those skilled in the art. Although the present disclosure has been described by using specific preferred embodiments, it should be understood that the claimed disclosure should not be unduly limited to these specific embodiments. In fact, many variations of the described modes for carrying out the disclosure that are obvious to those skilled in the art are intended to be included within the scope of the appended claims.

**Claims**

1.   A method of treating membranous nephropathy, comprising administering a therapeutically effective amount of a TACI-Fc fusion protein to a patient with membranous nephropathy, wherein the TACI-Fc fusion protein comprises:

   (i) a TACI extracellular region or a fragment thereof binding to Blys and/or APRIL; and
   (ii) a fragment of human immunoglobulin constant region.

2.   The method according to claim 1, wherein the TACI extracellular region or the fragment thereof binding to Blys and/or APRIL comprises an amino acid sequence set forth in SEQ ID NO: 1.

3.   The method according to claim 2, wherein the human immunoglobulin is IgG1, or the fragment of human immunoglobulin constant region comprises an amino acid sequence set forth in SEQ ID NO: 2 or an amino acid sequence having at least 90%, at least 91%, at least 92%, at least 93%, at least 95%, at least 96%, at least 97%, at least 98% or at least 99% identity to SEQ ID NO: 2.

4.   The method according to claim 3, wherein the fragment of human immunoglobulin constant region comprises one or more modifications of amino acid at positions 3, 8, 14, 15, 17, 110, 111 or 173 of SEQ ID NO: 2.

5.   The method according to claim 4, wherein the modification is substitution, deletion or insertion of an amino acid.

6.   The method according to claim 5, wherein the substitution is selected from the group consisting of P3T, L8P, L14A, L15E, G17A, A110S, P111S and A173T.

7.   The method according to claim 7, wherein the fragment of human immunoglobulin constant region comprises an amino acid sequence set forth in SEQ ID NO: 3.

8. The method according to claim 1, wherein the TACI-Fc fusion protein has an amino acid sequence set forth in SEQ ID NO: 4.

9. The method according to claim 8, wherein the TACI-Fc fusion protein is Telitacicept.

10. The method according to claim 8 or 9, wherein the membranous nephropathy includes primary membranous nephropathy or secondary membranous nephropathy.

11. The method according to claim 10, wherein membranous nephropathy is expressed as PLA2R positive or PLA2R negative.

12. The method according to claim 11, wherein the patient is an adult patient or a child patient.

13. The method according to claim 12, wherein the patient has previously received a treatment regimen for membranous nephropathy.

14. The method according to claim 12 or 13, wherein the TACI-Fc fusion protein is administered at a single dose of about 0.1 to 10 mg/kg.

15. The method according to claim 12 or 13, wherein the TACI-Fc fusion protein is administered at a single dose of 160 to 240 mg, further preferably 160 mg or 240 mg.

16. The method according to claim 12 or 13, wherein the TACI-Fc fusion protein is administered subcutaneously, intramuscularly or intravenously, or at a thigh, abdomen or upper arm.

17. The method according to claim 12 or 13, wherein the TACI-Fc fusion protein is administered 2-4 times at an interval of one month and/or the administration lasts for about 2 to 50 weeks .

18. The method according to claim 17, wherein the TACI-Fc fusion protein is administered at a frequency of once a week.

FIG. 1

FIG. 2

FIG. 3

FIG. 4

## INTERNATIONAL SEARCH REPORT

| International application No. |
|---|
| **PCT/CN2023/122388** |

### A. CLASSIFICATION OF SUBJECT MATTER

A61K 47/68(2017.01)i; A61K38/17(2006.01)i; C07K19/00(2006.01)i; A61K39/395(2006.01)i; A61K35/22(2015.01)i; A61P13/12(2006.01)i

According to International Patent Classification (IPC) or to both national classification and IPC

### B. FIELDS SEARCHED

Minimum documentation searched (classification system followed by classification symbols)

IPC: A61K C07K A61P

Documentation searched other than minimum documentation to the extent that such documents are included in the fields searched

Electronic data base consulted during the international search (name of data base and, where practicable, search terms used)

CNABS; CNTXT; DWPI; VEN; ENTXTC; ENTXT; WPABS; CNKI; VCN; CJFD; 万方, WANFANG; ISI Web of Science; 读秀学术, Duxiu Scholar; 中国专利生物序列检索数据库, China Patent Biological Sequence Search Database; Springer; NCBI; ELiviser; STNext: 荣昌生物, 膜性肾病, 特发性膜性肾病, 泰它西普, 泰他西普, 泰爱, 膜性, 膜型, 肾, 增殖诱导配体, B淋巴细胞刺激因子, 序列检索, sequence search, membranous nephropathy, MN, idiopathic membranous nephropathy , IMN, Telitacicept, RC18, membranous, kidney, TACI-Fc, APRIL, BLyS, PLA2R

### C. DOCUMENTS CONSIDERED TO BE RELEVANT

| Category* | Citation of document, with indication, where appropriate, of the relevant passages | Relevant to claim No. |
|---|---|---|
| PX | WANG, Jinling et al. "Anti-phospholipase A2 Receptor-associated Membranous Nephropathy with Human Immunodeficiency Virus Infection Treated with Telitacicept: A Case Report" *World Journal of Clinical Cases*, Vol. 11, No. (22), 06 August 2023 (2023-08-06), pp. 5309-5315<br>p. 5309, abstract | 1-18 |
| X | 医学慕课 (Medicalmooc). "例践标杆(2)\|膜性狼疮肾炎患者治疗棘手, 生物制剂泰它西普有效! (Non-official translation: Practice benchmarking (2)\|Treatment of Patients with Membranous Lupus Nephritis is Tough, and Biological Preparation Telitacicept is Effective!)" 微信公众号 *(Wechat public account)*, 17 September 2021 (2021-09-17), pp. 1-12<br>page 3, paragraph 1, page 8, Adjustment of medication regimen, page 9, Curative effect, page 10, Subsequent maintenance therapy, and page 12, paragraph 2 | 1-18 |
| A | CN 101628111 A (ZYMOGENETICS INC.) 20 January 2010 (2010-01-20)<br>description, page 4, paragraph 2, and page 5, paragraph 4 | 1-18 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 January 2024** | **12 January 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/122388**

## C.    DOCUMENTS CONSIDERED TO BE RELEVANT

| Category* | Citation of document, with indication, where appropriate, of the relevant passages | Relevant to claim No. |
|---|---|---|
| A | CN 113573732 A (REMEGEN BIOPHARMACEUTICAL (YANTAI) CO., LTD.) 29 October 2021 (2021-10-29)<br>claims 1-27 | 1-18 |
| A | US 2010136008 A1 (FANG JIANMIN; LIU ZHENG;) 03 June 2010 (2010-06-03)<br>claims 1-23 | 1-18 |
| A | Benjamin Y.F. So et al. "B Cells in Primary Membranous Nephropathy: Escape from Immune Tolerance and Implications for Patient Management"<br>*International Journal of Molecular Sciences,* 17 December 2021 (2021-12-17), pp. 1-19<br>abstract | 1-18 |

Form PCT/ISA/210 (second sheet) (July 2022)

## INTERNATIONAL SEARCH REPORT

International application No.

**PCT/CN2023/122388**

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    ☑   forming part of the international application as filed.

     b.    ☐   furnished subsequent to the international filing date for the purposes of international search (Rule 13*ter*.1(a)),

            ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT

International application No.

**PCT/CN2023/122388**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.:
because they relate to subject matter not required to be searched by this Authority, namely:

The subject matter of claims 1-18 relates to a method for treating membranous nephropathy. However, the ultimate purpose of the above-mentioned method is to treat diseases in a living human or animal body, which falls within the scope of disease diagnosis and treatment methods as defined in PCT Rule 39.1(4).

With regard to claim 1, the reasonably expected amendments are made as follows:

Claim 1: the use of a TACI-Fc fusion protein in the preparation of a drug for treating membranous nephropathy, wherein the TACI-Fc fusion protein comprises:...

Dependent claims 2-18: the use according to claim..., ...

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

INTERNATIONAL SEARCH REPORT
Information on patent family members

International application No.

**PCT/CN2023/122388**

| Patent document cited in search report | | | Publication date (day/month/year) | Patent family member(s) | | | Publication date (day/month/year) |
|---|---|---|---|---|---|---|---|
| CN | 101628111 | A | 20 January 2010 | HRP | 20030948 | A2 | 30 June 2004 |
| | | | | HRP | 20030948 | B1 | 30 June 2013 |
| | | | | SI | 1436003 | T1 | 26 February 2010 |
| | | | | US | 2011229473 | A1 | 22 September 2011 |
| | | | | US | 8524232 | B2 | 03 September 2013 |
| | | | | EA | 200600894 | A1 | 31 August 2007 |
| | | | | EA | 010594 | B1 | 30 October 2008 |
| | | | | MXPA | 03010687 | A | 01 July 2004 |
| | | | | DK | 1436003 | T3 | 15 March 2010 |
| | | | | US | 2003103986 | A1 | 05 June 2003 |
| | | | | EA | 200301146 | A2 | 24 June 2004 |
| | | | | EA | 200301146 | A3 | 24 February 2005 |
| | | | | EA | 007275 | B1 | 25 August 2006 |
| | | | | IL | 158920 | A0 | 12 May 2004 |
| | | | | NO | 20035173 | D0 | 21 November 2003 |
| | | | | NO | 20035173 | L | 23 January 2004 |
| | | | | NO | 337295 | B1 | 07 March 2016 |
| | | | | US | 2006034852 | A1 | 16 February 2006 |
| | | | | US | 7501497 | B2 | 10 March 2009 |
| | | | | KR | 20090080570 | A | 24 July 2009 |
| | | | | KR | 100976743 | B1 | 19 August 2010 |
| | | | | EP | 1436003 | A2 | 14 July 2004 |
| | | | | EP | 1436003 | A4 | 27 April 2005 |
| | | | | EP | 1436003 | B1 | 28 October 2009 |
| | | | | EP | 1436003 | B3 | 14 March 2012 |
| | | | | SI | 2116259 | T1 | 30 November 2012 |
| | | | | ES | 2334772 | T3 | 16 March 2010 |
| | | | | ES | 2334772 | T7 | 19 November 2012 |
| | | | | UA | 82830 | C2 | 26 May 2008 |
| | | | | US | 2010183609 | A1 | 22 July 2010 |
| | | | | US | 7964711 | B2 | 21 June 2011 |
| | | | | JP | 2009232856 | A | 15 October 2009 |
| | | | | JP | 5149245 | B2 | 20 February 2013 |
| | | | | HK | 1137659 | A1 | 06 August 2010 |
| | | | | KR | 20040030628 | A | 09 April 2004 |
| | | | | PL | 366760 | A1 | 07 February 2005 |
| | | | | PL | 219013 | B1 | 27 February 2015 |
| | | | | US | 2010129384 | A1 | 27 May 2010 |
| | | | | US | 7862814 | B2 | 04 January 2011 |
| | | | | ZA | 200308984 | B | 20 July 2004 |
| | | | | HRP | 20130413 | A2 | 31 July 2013 |
| | | | | US | 2014328844 | A1 | 06 November 2014 |
| | | | | US | 9346878 | B2 | 24 May 2016 |
| | | | | YU | 92503 | A | 25 May 2006 |
| | | | | RS | 52228 | B | 31 October 2012 |
| | | | | ES | 2379977 | T3 | 07 May 2012 |
| | | | | EP | 2116259 | A1 | 11 November 2009 |
| | | | | EP | 2116259 | B1 | 25 January 2012 |
| | | | | IL | 217265 | A0 | 29 February 2012 |
| | | | | IL | 217265 | A | 30 May 2013 |

Form PCT/ISA/210 (patent family annex) (July 2022)

**INTERNATIONAL SEARCH REPORT**
Information on patent family members

International application No.

**PCT/CN2023/122388**

| Patent document cited in search report | | | Publication date (day/month/year) | Patent family member(s) | | | Publication date (day/month/year) |
|---|---|---|---|---|---|---|---|
| | | | | MEP | 21708 | A | 10 June 2010 |
| | | | | CY | 1109751 | T1 | 10 September 2014 |
| | | | | AU | 2002305646 | B2 | 04 September 2008 |
| | | | | AU | 2002305646 | C1 | 06 August 2009 |
| | | | | PT | 1436003 | E | 12 March 2010 |
| | | | | US | 2009209006 | A1 | 20 August 2009 |
| | | | | US | 7635767 | B2 | 22 December 2009 |
| | | | | CA | 2448123 | A1 | 28 November 2002 |
| | | | | CA | 2448123 | C | 11 September 2012 |
| | | | | DK | 2116259 | T3 | 21 May 2012 |
| | | | | WO | 02094852 | A2 | 28 November 2002 |
| | | | | WO | 02094852 | A8 | 22 April 2004 |
| | | | | RS | 20120253 | A1 | 28 February 2013 |
| | | | | BR | 0209933 | A | 30 March 2004 |
| | | | | BRPI | 0209933 | B1 | 16 October 2018 |
| | | | | BRPI | 0209933 | B8 | 25 May 2021 |
| | | | | JP | 2004535182 | A | 25 November 2004 |
| | | | | HK | 1076603 | A1 | 20 January 2006 |
| | | | | DE | 60234202 | D1 | 10 December 2009 |
| | | | | CY | 1112840 | T1 | 10 February 2016 |
| | | | | ATE | 446771 | T1 | 15 November 2009 |
| | | | | ATE | 542545 | T1 | 15 February 2012 |
| | | | | PL | 403488 | A1 | 08 July 2013 |
| | | | | US | 2010130728 | A1 | 27 May 2010 |
| | | | | US | 7951919 | B2 | 31 May 2011 |
| | | | | KR | 20100061860 | A | 09 June 2010 |
| | | | | KR | 101021124 | B1 | 14 March 2011 |
| | | | | PT | 2116259 | E | 09 April 2012 |
| | | | | NZ | 529638 | A | 31 August 2007 |
| | | | | US | 2013309231 | A1 | 21 November 2013 |
| | | | | US | 8815238 | B2 | 26 August 2014 |
| CN | 113573732 | A | 29 October 2021 | None | | | |
| US | 2010136008 | A1 | 03 June 2010 | WO | 2008154814 | A1 | 24 December 2008 |
| | | | | JP | 2010529967 | A | 02 September 2010 |
| | | | | JP | 5372917 | B2 | 18 December 2013 |
| | | | | KR | 20100009600 | A | 27 January 2010 |
| | | | | KR | 101204229 | B1 | 26 November 2012 |
| | | | | US | 8193316 | B2 | 05 June 2012 |
| | | | | RU | 2009148020 | A | 10 July 2011 |
| | | | | RU | 2433141 | C2 | 10 November 2011 |
| | | | | BRPI | 0811333 | A2 | 16 June 2015 |
| | | | | BRPI | 0811333 | B1 | 15 September 2020 |
| | | | | BRPI | 0811333 | B8 | 25 May 2021 |
| | | | | EP | 2161287 | A1 | 10 March 2010 |
| | | | | EP | 2161287 | A4 | 11 August 2010 |
| | | | | EP | 2161287 | B1 | 04 March 2015 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 969102 A
- US 6316222 B
- US 6500428 B
- US 569245
- US 09627206 B
- CN 101323643 A

**Non-patent literature cited in the description**

- **ALSHARHAN L** ; **BECK LH JR.** ; **2021 MAR**. Membranous Nephropathy: Core Curriculum 2021. *Am J Kidney Dis.*, vol. 77 (3), 440-453
- *J. biol. chem*, 1968, vol. 243, 3558